# EUROPEAN PATENT SPECIFICATION

(11) **EP 3 490 624 B1**
(45) Date of publication and mention of the grant of the patent: **09.02.2022**
(21) Application number: 17762214.9
(22) Date of filing: 01.08.2017
(51) Int. Cl.: A61L 27/22, A61L 27/52, A61L 27/54, A61L 27/56

(54) **NERVE GUIDANCE CONDUITS, METHODS OF PRODUCTION AND USES THEREOF**
NERVENLEITUNGSKANÄLE, VERFAHREN ZUR HERSTELLUNG DAVON UND VERWENDUNGEN DAVON
CONDUITS DE GUIDAGE DE NERFS, LEURS PROCÉDÉS DE PRODUCTION ET LEURS UTILISATIONS

(30) Priority: 01.08.2016 PT 2016109562
(43) Date of publication of application: 05.06.2019
(73) Proprietor: Association for the Advancement of Tissue Engineering and Cell based Technologies & Therapies (A4TEC) - Associação, 4710-057 Braga (PT)
(72) Inventor: RODRIGUES DE CARVALHO, Cristiana, 4805-549 Vermil (PT); BEBIANO E COSTA, João Pedro, 5000-717 Vila Real (PT); PINTO RIBEIRO, Viviana, 5450-009 Vila Pouca de Aguiar (PT); DA SILVA CORREIA DE OLIVEIRA, Joana Catarina, 4715-178 Braga (PT); ANTUNES DE OLIVEIRA, Joaquim Miguel, 4715-178 Braga (PT); GONÇALVES DOS REIS, Rui Luís, 4250-242 Porto (PT)
(74) Representative: Patentree
(86) International application number: PCT/IB2017/054708
(87) International publication number: WO 2018/025186

(56) References cited:
- CA-A1- 2 580 349
- DATABASE CA [Online] CHEMICAL ABSTRACTS SERVICE, COLUMBUS, OHIO, US; YAN, LEPING ET AL: "Silk-fibroin hydrogels for medical applications", XP002775238, retrieved from STN Database accession no. 2013:1642701 & YAN, LEPING ET AL: "Silk-fibroin hydrogels for medical applications", PORT. PAT. APPL., 79PP. CODEN: PTXXB9, 2013,
- BENJAMIN P. PARTLOW ET AL: "Highly Tunable Elastomeric Silk Biomaterials", ADVANCED FUNCTIONAL MATERIALS, vol. 24, no. 29, 1 August 2014 (2014-08-01), pages 4615-4624, XP55420680, DE ISSN: 1616-301X, DOI: 10.1002/adfm.201400526
- NAZAROV RINA ET AL: "Porous 3-D scaffolds from regenerated silk fibroin", BIOMACROMOLECULES, AMERICAN CHEMICAL SOCIETY, vol. 5, no. 3, 1 May 2004 (2004-05-01), pages 718-726, XP002439711, ISSN: 1525-7797, DOI: 10.1021/BM034327E
- H.-J. JIN ET AL: "Water-Stable Silk Films with Reduced [beta]-Sheet Content", ADVANCED FUNCTIONAL MATERIALS, vol. 15, no. 8, 1 July 2005 (2005-07-01), pages 1241-1247, XP55093334, ISSN: 1616-301X, DOI: 10.1002/adfm.200400405
- YAN, LEPING ET AL: "Silk-fibroin hydrogels for medical applications", PORT. PAT. APPL., 79PP. CODEN: PTXXB9, 2013,
- Benjamin P. Partlow ET AL: "Highly Tunable Elastomeric Silk Biomaterials", Advanced Functional Materials, vol. 24, no. 29, 1 August 2014 (2014-08-01), pages 4615-4624, XP055420680, DE ISSN: 1616-301X, DOI: 10.1002/adfm.201400526
- NAZAROV RINA ET AL: "Porous 3-D scaffolds from regenerated silk fibroin", BIOMACROMOLECULES, AMERICAN CHEMICAL SOCIETY, US, vol. 5, no. 3, 1 May 2004 (2004-05-01), pages 718-726, XP002439711, ISSN: 1525-7797, DOI: 10.1021/BM034327E
- H.-J. JIN ET AL: "Water-Stable Silk Films with Reduced [beta]-Sheet Content", ADVANCED FUNCTIONAL MATERIALS, vol. 15, no. 8, 1 July 2005 (2005-07-01), pages 1241-1247, XP055093334, ISSN: 1616-301X, DOI: 10.1002/adfm.200400405

## Description

### Technical field

The present disclosure relates to a silk fibroin tubular conduit.

The tubular conduit of the present disclosure may be use in treatment of diseases that involve the repair and/or regeneration of tissues, nerves or bone. In particular, the use for peripheral nerve regeneration.

### Background

Document CA 2580349 described methods and apparatus for enhanced growth of peripheral nerves and nervous tissue. The device has a matrix is silk fibroin protein, the matrix was cross-linked using formaldehyde gas, citrate ions, ribose, glyoxal or genipin. However CA 2580349 does not describe an enzymatic cross-linking that allows obtaining a hydrogel as an intermediate step of the process of fabrication of the tubular conduit that confers advantages. In addition, by the methodology now disclosed, it is obtained a uniform device, in comparison to the composite material comprising different components that is achieved in the patent CA 2580349.

Document WO 2009023615 A1 describes a tubular silk fibroin compositions that can be used in the repair or replacement of damaged or diseased blood vessels. However, the WO 2009023615 A1 does not disclose the use of tubular silk compositions for other applications other than that of repair or replacement of damaged or diseased blood vessels. The present disclosure is intended for peripheral nerve regeneration. In addition, the methodology described in the patent referred above consists in a technique called dipping or spraying. Therefore, the result of this process is a layer-by-layer obtained device. In contrast, the present disclosure produces a uniform and one layered device. Furthermore, with the present disclosure, it is possible to control and fine-tune the porosity of tubular device by adjusting parameters in the process and in the referred patent the authors have to add different compounds such as polyethylene oxide (PEO).

Document CN 101879330 B discloses a small-calibre silk fibroin tubular material. The document uses the fibroin solution impregnation, drying, curing and other methods can close integration of three layers of material, and the use of heparin and other anti-clotting drugs for its inner processed material has anticoagulant properties, it can be used to repair blood vessels, nerves, and other tissues. Nevertheless, this document does not disclosed the attainment of a hydrogel during the process, due to an enzymatic cross-linking obtained with horseradish peroxidase. Furthermore, the method of production now disclosed is related to mould casting, as the one mentioned in the patent refers to the electrospinning technique, in which is mandatory to use aggressive and not biocompatible reagents such as formic acid and hexafluoroisopropanol. The mentioned device implicates the presence of three layers, mimicking the natural structure of blood vessels and the use of high temperatures, none of which is present in our methodology since it is green and cell-friendly.

US 9068282 B2 described a system and method for making biomaterial structures that can be used as tubular vessels for tissue engineering. However, US 9068282 B2 does not disclosed a mould casting technique that makes use of a hydrogel as an intermediate component of the process to produce a tubular device.

A bibliographic research revealed many publications related to the production of silk nerve guidance conduits, but mainly by electrospinning and dipping in silk aqueous solutions.

Benjamin P. Partlow ET AL: "Highly Tunable Elastomeric Silk Biomaterials", Advanced Functional Materials, vol. 24, no. 29, 1 August 2014, pages 4615-4624 discloses a method to fabricate an enzymatically cross-linked fibroin hydrogel using horseradish peroxidase and hydrogen peroxide.

Unsatisfying functional recovery after peripheral nerve injury (PNI) is still a significant clinical challenge faced today, even after decades of research in the field (1). PNIs only are related to 8.5 million restricted activity days and almost 5 million "bed days" each year around the world (2). Consequently, over 200,000 peripheral nerve repair procedures are performed annually in the US alone (3). A variety of reasons may lead to PNI, such as car accidents, military and sport injuries as well as degenerative diseases. Although peripheral nervous system (PNS) has a superior capacity to regenerate when compared to CNS, they repeatedly result in painful neuropathies owing to reduction in motor function and sensory perception. Surgical interventions such as neurorrhaphy, which is the direct suture repair without the use of grafted materials - may be used in cases where a short (<5 mm) nerve gap has to be overcome. However, larger defects repaired by neurorrhaphy, exhibit excessive tension over the suture line and offer poor surgical results (4). To this day, the typical choice in this situation is a nerve autograft that is harvested from another site in the body. However, this recognized "gold standard" technique for peripheral nerve repair is limited by tissue availability, donor-site morbidity, secondary deformities, as well as potential differences in tissue structure and size. In the last 30 years, FDA has approved a few devices based on natural and synthetic biomaterials to repair nerve defects arising from PNI, since autograft is not always an option. The effort of the scientific community to tackle this issue is visible when one considers the 732 publications in 2015 alone concerning peripheral nerve regeneration. Still, despite all of this research and effort, not many alternatives reach the market or when they do, they do not outperform the autograft.

The first materials used in nerve guidance conduits were synthetic, such as PCL, PLGA and polyurethane. Among the natural and biodegradable materials, agarose, chitosan, collagen, fibrin, gelatine, keratin and silk have been used.

These facts are disclosed in order to illustrate the technical problem addressed by the present disclosure.

### General description

The scope of protection is defined by the claims.

The present disclosure relates to a silk fibroin tubular conduit according to claims 1-13 and to a composition of silk fibroin hydrogel for use in the treatment of peripheral nerve injury, a spinal cord injury, regeneration of peripheral nerve cells, brain diseases or nerve degenerative diseases according to claims 14-15. In particular, the use for peripheral nerve regeneration.

Materials used for peripheral nerve regeneration, requires not only to have good biocompatibility, but must also have a controllable biodegradability, certain mechanical strength, and performance can be related to the release of drugs or other factors.

There are three methods for producing silk tubular structures: mould casting, dipping and electrospinning. The method now disclosed is included in the mould casting. However, the process now disclosed is different from the ones of the state of the art. In the process of the state of the art, an aqueous silk solution is made and injected in the mould and β-sheet is induced, using a standardized method comprising a methanol solution for induction of β-sheet crystalline structure, transforming a transparent solution into opaque and solid final material.

The present disclosure comprises the transformation of an initially produced aqueous silk solution into an amorphous and transparent hydrogel, through a peroxidase-mediated cross-link reaction.

In an embodiment, surprisingly a temporary β-sheet is induced with liquid nitrogen facilitating the removal of the outer mould and a posterior permanent semicrystalline structure is induced with an ethanol solution. Alternatively, the induction of β-sheet conformation can be made by freezing at -80°C and freeze-drying may be performed to avoid the use of organic solvents.

In an embodiment, silk fibroin may contain around 5 mol% tyrosine groups, which are oxidized by peroxidase/hydrogen peroxide and subsequently cross-linked to form a three dimensional network. Silk fibroin hydrogels are achieved by the cross-linking of tyrosine groups in silk fibroin. This cross-link leads to a stronger and more stable three-dimensional network, thus conferring the scaffold higher mechanical properties, more elasticity and a lower degradation rate, when compared to tubes that did not undergo this cross-link before turning in β-sheet conformation.

In an embodiment, by transforming the aqueous solution in a hydrogel in the middle of the process, increasing it viscosity without having to increase silk concentration, it is easier to incorporate and making gradients of bioactive molecules. Therefore, the present disclosure relates to a more stable three-dimensional network after the formation of the hydrogel, as the swelling shall not be as intense as a tube produced without the formation of the hydrogel, which is an advantage for PNR. With this methodology, it is possible to produce tubular conduits/tubes with variable length, variable wall thickness and variable intraluminal diameter.

The tubular conduit according to the present disclosure provides a fibroin silk tubular conduit with improved mechanical properties. This allows implanting the products obtainable by the present process in vivo into regions where they are exposed to specific mechanical influences, such as sciatic nerve or hand, wrist and forearms regions.

In an embodiment, in vivo implants of a nerve conduit may be exposed to pressure forces, which may cause collapse of said implants. In such a case, a tube of silk fibroin would collapse and hinder a growing nerve covered by such tube.

In an embodiment, the fibroin silk tubular conduit obtainable by the process disclosed in the present subject matter allows provision of transplants resisting such forces causing crush or contusion.

In an embodiment, the process now disclosed for obtain a fibroin silk tubular conduit, results in a tubular conduit with improved properties, since as the hydrogel is obtained during said process, which will comprise a more stable and stronger three-dimensional network. This step provides advantages in terms of mechanical features, more elasticity, kinking-resistance ability, adjustable permeability and a more controlled degradation and swelling rate.

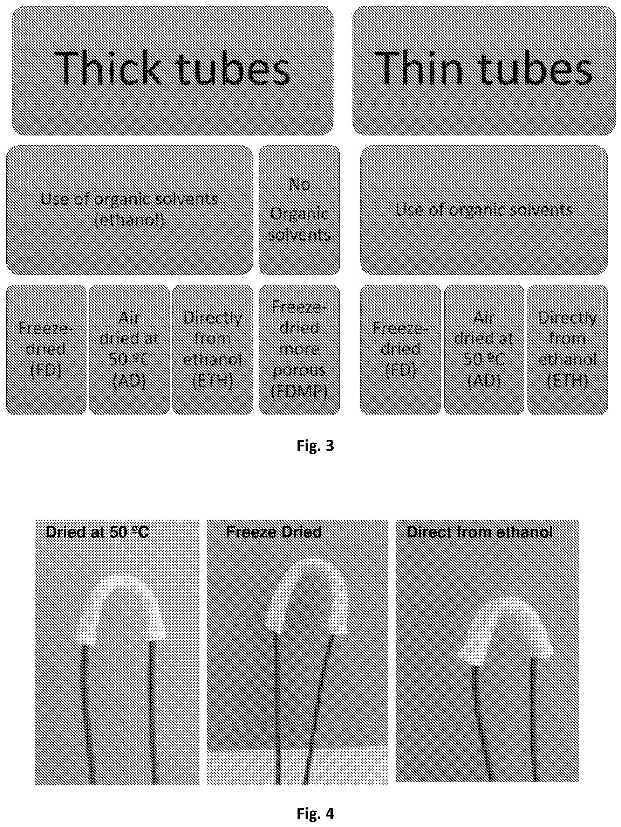

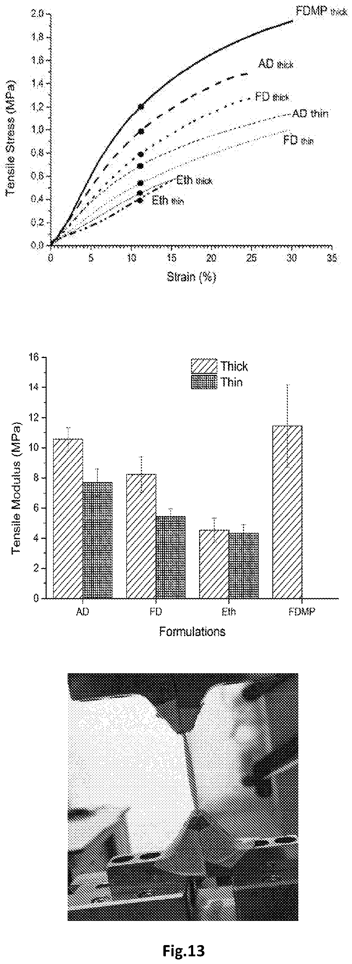

The tubular conduit described in the present subject-matter has improved elastic proprieties, kinking resistant and shape recovery as show in fig.4 and 13.

Thus, the tubular conduit discloses in the present disclosure advantageously regenerates nerve cells and blood vessels in peripheral nerve tissues and thus rehabilitates the injured nerve tissues, improve nerve conduction velocity, and relief of pain induced by traumatic peripheral nerve injury.

An aspect of the present disclosure is related to a tubular conduit comprising an enzymatically cross-linked silk fibroin hydrogel wherein said tube is predominantly β-sheet conformation and comprises a porosity from 1 to 70% and a pore size from 1 to 10 µm, optionally the pore may be interconnected.

The tubular conduit comprises porosity from 1 to 70% and a pore size from 1 to 7 µm, optionally the pore may be interconnected.

In an embodiment for better results, the porosity may be 3-25%, preferably 5-10%.

In an embodiment for better results, the pore size may be 1-7 µm.

In an embodiment for better results, the tubular conduit may have a wall with a thickness of 0.2 mm - 2 mm, preferably 0.3 mm - 1 mm, more preferably 0.3 mm - 0.7 mm.

In an embodiment for better results, the cross-linked silk fibroin hydrogel may be enzymatically cross-linked with horseradish peroxidase and hydrogen peroxide.

In an embodiment for better results, the hydrogel now disclosed may comprise between 5-25% (wt %) of silk fibroin, preferably between 12-18% (wt %) of silk fibroin, more preferably between 15-16% (wt %) of silk fibroin.

In an embodiment for better results, the tubular conduit may comprise an internal diameter of 1 mm - 10 mm, preferably 2 mm - 4 mm.

In an embodiment for better results, the tubular conduit may have a length of 5 mm - 100 mm, preferably 10 mm - 50 mm.

In an embodiment for better results, the tubular conduit of the present disclosure may further comprises a conductive agent, preferably selected form a list consisting of: gold, silver, polypirrole, or mixtures thereof.

In an embodiment for better results, the tubular conduit may further comprise hyaluronic acid, alginate, casein, polyethylene oxide, polyethylene glycol, collagen, fibronectin, keratin, polyaspartic acid, polylysine, chitosan, pectin, polylactic acid, polycaprolactone, polyglycolic acid, polyhydroxyalkanoate, polyanhydride, and mixtures thereof.

In an embodiment for better results, the tubular conduit may further comprise a biological active agent, a therapeutic agent, an additive, a pharmaceutically acceptable excipient, a pharmaceutically acceptable carrier, and mixtures thereof.

In an embodiment for better results, the biologically active agent may be selected from the following list: a peptide, a protein, a nucleic acid, an antibody, an aptamer, an anticoagulant agent, a growth factor, a cytokine, an antibiotic, an immunosuppressor, a steroid, non-steroidal anti-inflammatory drug, and mixtures thereof. It is understood that other drugs or factors to promote nerve regeneration or to suppress the formation of glioma or fibrosis can be added.

In an embodiment for better results, the thickness of the walls of the final conduits can be modified by changing the specific sizes of the moulds used. For more permeable conduits, thin wall conduits must be chosen.

In an embodiment for better results, the tubular conduit may comprise of dorsal root ganglia, Schwann cells and/or stem cells. Other cell types could also be added as required.

The present disclosure also relates to a composition of silk fibroin hydrogel enzymatically cross-linked, in particular with horseradish peroxidase and hydrogen peroxide,
for use in the treatment of peripheral nerve injury, a spinal cord injury, regeneration of peripheral nerve cells, brain diseases or nerve degenerative diseases;
wherein said composition is administrated in the form of a tubular conduit;
wherein said tubular conduit comprises a β-sheet conformation obtainable by an enzymatically cross-linked of silk fibroin hydrogel; comprising a porosity from 1 to 70% and a pore size from 1 to 10 µm.

In an embodiment for better results, the tubular conduit may be for use in the treatment of traumatic peripheral nerve injury.

In an embodiment for better results, the tubular conduit may comprise dorsal root ganglia and/or Schwann cells.

In an embodiment, final β-sheet conformation may be determined by FTIR, NanoIR and XRD techniques, to assess the presence of functional groups and the crystallinity of final material.

In an embodiment, the porosity of the tubular conduit may be determined by Micro-CT 3D reconstructions in which morphometric parameters such as total % of porosity, mean pore size and trabecular thickness will be quantified.

In an embodiment, the permeability according to the pore sizes may be assessed with fluorescent dyed molecules quantification or O2 permeability studies.

In an embodiment, the pore size distribution of the conduit may be determined by Micro-CT 3D analysis and SEM micrographs.

In an embodiment, the pores may be interconnected.

### Brief description of the drawings

The following figures provide preferred embodiments for illustrating the description and should not be seen as limiting the scope of disclosure.
Figure 1. Schematic representation of the preparation of a tubular conduit/ nerve guidance conduit of silk fibroin.
Figure 2. Schematic representation of embodiment of a tubular conduit/nerve guidance conduit of silk fibroin after production.
Figure 3. Schematic representation of possible methods for obtain the tubular conduit described in the present disclosure.
Figure 4. Schematic representation of kinking resistance ability of thick wall conduits.

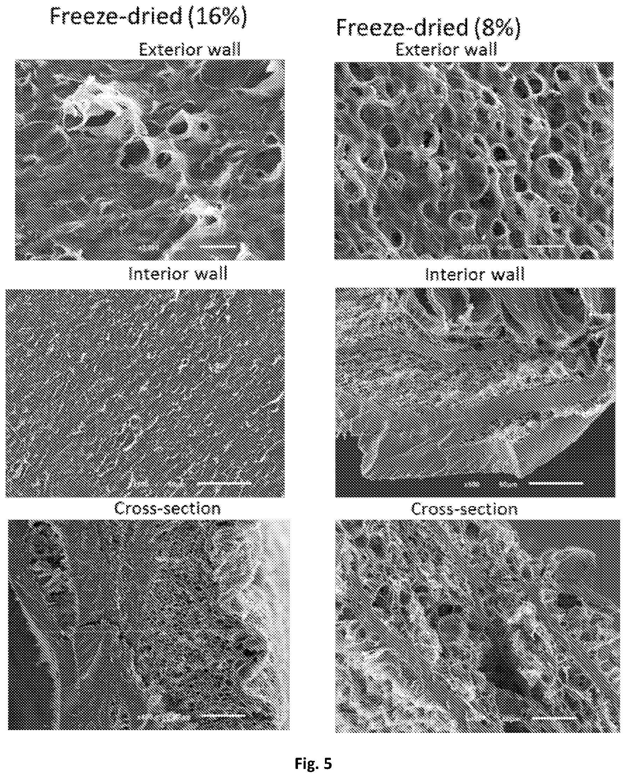

Figure 5. Schematic representation of SEM micrographs revealing the increased porosity achieved by reducing silk concentration from 16% to 8%. The cross-section is specially affected, with high interconnectivity from the inner to the outer walls of the conduit.
Figure 6. Schematic representation of: A) Stereomicroscope pictures of the different silk conduits obtained with different drying methods; B) SEM micrographs of the different silk conduits obtained with different drying methods that lead to different micro-structure and porosity.
Figure 7. Schematic representation of: A) 3D micro-ct reconstruction of thick and thin wall conduits obtained used different sized moulds; B) SEM micrographs of the mentioned conduits, where wall thickness varies; C) Stereomicroscope images of thick and thin wall conduits, in hydrated and dry state
Figure 8. Schematic representation of silk conduits containing gradients of bioactive molecules. A) Scheme of desired prototypes containing three different and increasing concentrations, from proximal to distal sites. B) Fabrication of a silk conduit (hydrogel step) with three different zones, that correspond to different growth factor concentrations. C) Different concentrations interface of obtained silk conduit.
Figure 9. Schematic representation of a simple hydrated silk conduit (white) when compared to a conductive silk - polypirrole conduit (black).

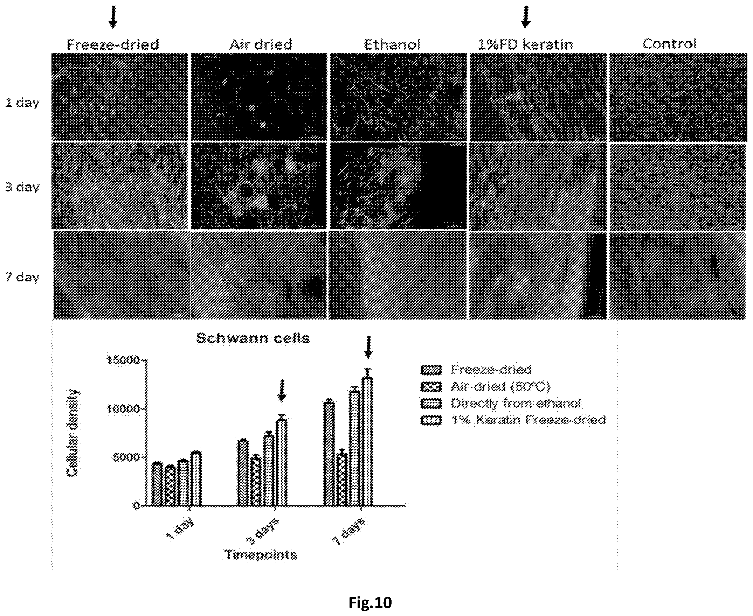

Figure 10. Schematic representation of a silk conduit when incorporating just 1% of hair keratin in the silk polymeric solution, cellular adhesion increased, which is proved qualitatively by Live/dead assay as well as quantitatively, by Alamar blue assay; (1%FD Keratin = freeze-dried conduit containing 1% keratin).
Figure 11. Schematic representation of the degradation profiles of several silk conduits formulations in the presence of 0,2U/ml of protease for 30 days. By varying only the method of drying, weight loss *in vivo* and *in vitro* will be modified; (FD= freeze-dried; AD= air dried at 50 °C; Eth= Directly from ethanol; FDMP= Freeze-dried more porous.

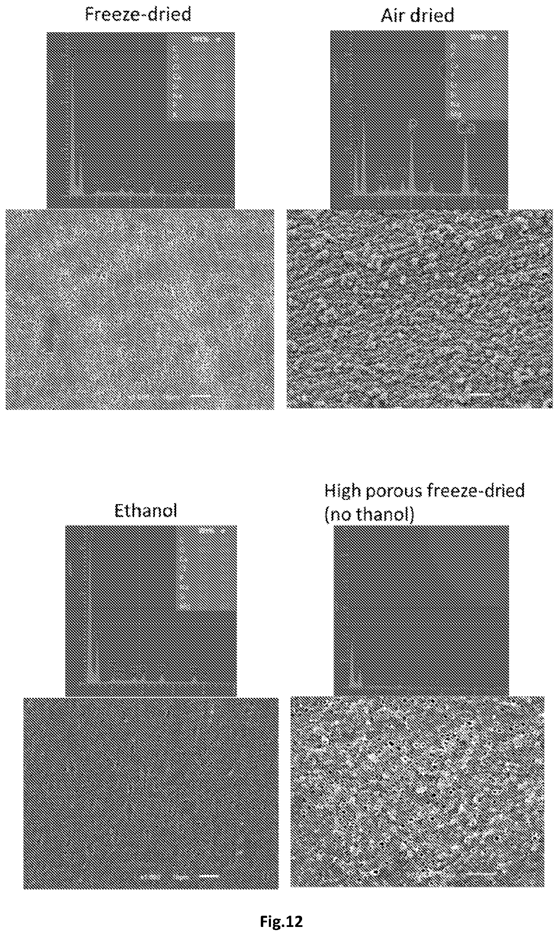

Figure 12. SEM micrographs and respective EDS spectra of the silk conduits after 30 days in Simulated Body Fluid. The Air Dried formulation was the only one that presented bioactivity, corresponding to the detection of calcium phosphates by EDS and visualization of cauliflower crystals in SEM micrographs.
Figure 13. Schematic representation of mechanical properties of some embodiment of the silk conduits: Tensile stress and Tensile modulus; ((FD= freeze-dried; AD= air dried at 50 °C; Eth= Directly from ethanol; FDMP= Freeze-dried more porous)

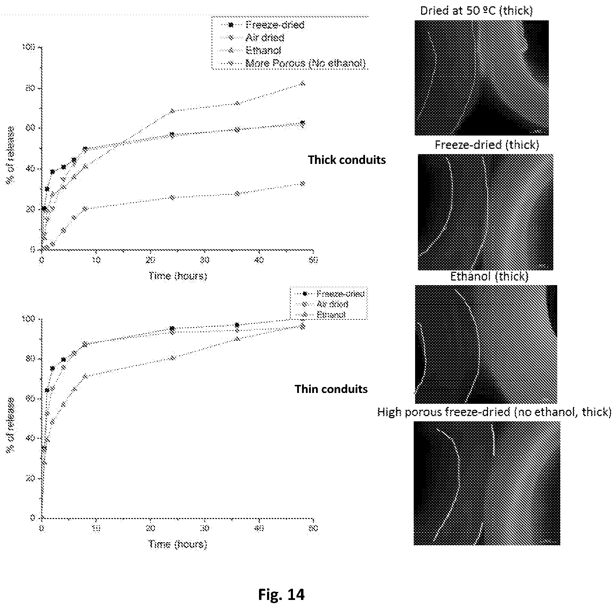

Figure 14. Differences in the silk conduits permeability of a 4kDa molecule due to the method of drying and due to the thickness of the conduit's wall.

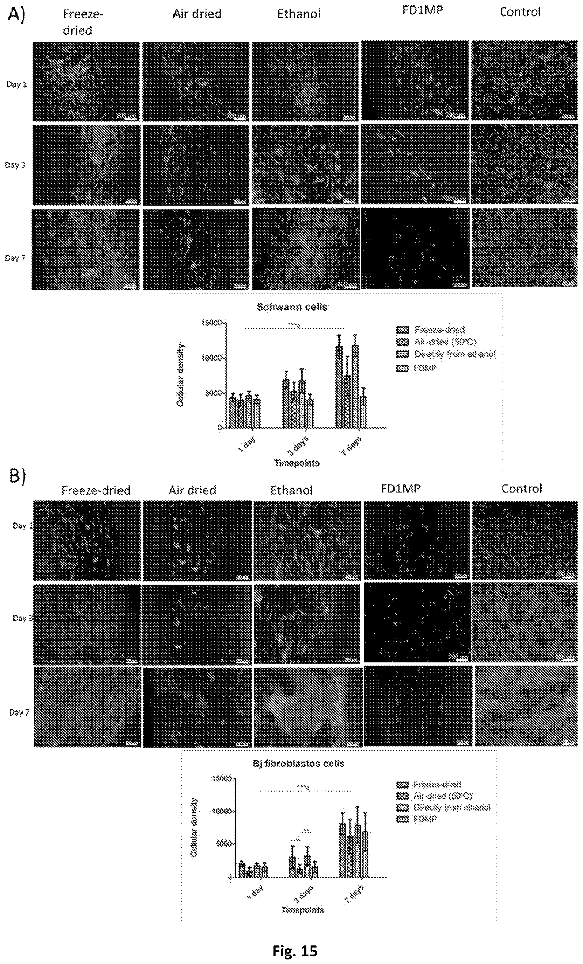

Figure 15. Schematic representation of: A) Results of Alamar Blue cellular density quantification and Live/dead qualitative assay after Schwann cells were seeded in all formulations of thick wall tubes after 7 days. B) Results of Alamar Blue cellular density quantification and Live/dead qualitative assay after BJ skin fibroblasts were seeded in all formulations of thick wall tubes after 7 days; (FDMP = Freeze-dried more porous).

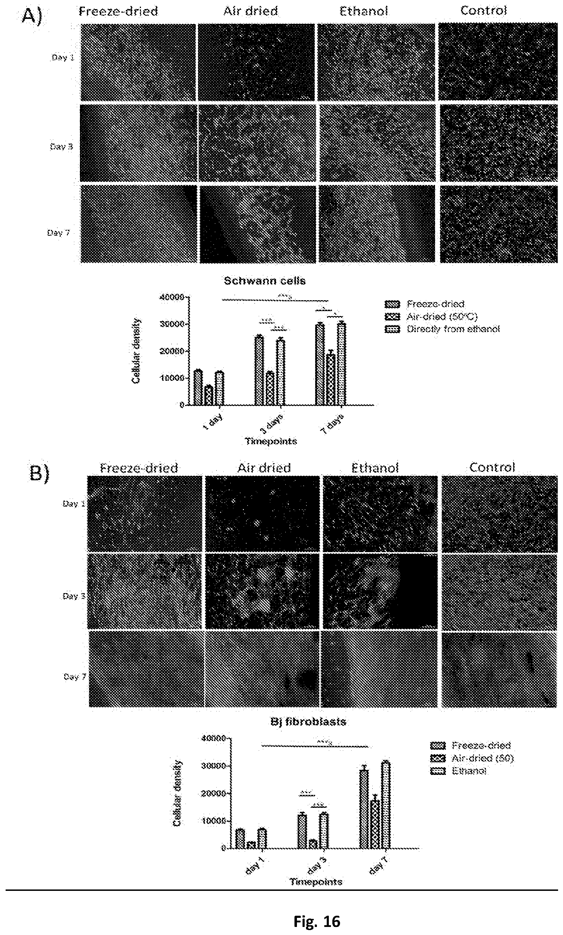

Figure 16. Schematic representation of: A) A) Results of Alamar Blue cellular density quantification and Live/dead qualitative assay after Schwann cells were seeded in all formulations of thick wall tubes after 7 days. B) Results of Alamar Blue cellular density quantification and Live/dead qualitative assay after BJ skin fibroblasts were seeded in all formulations of thick wall tubes after 7 days;

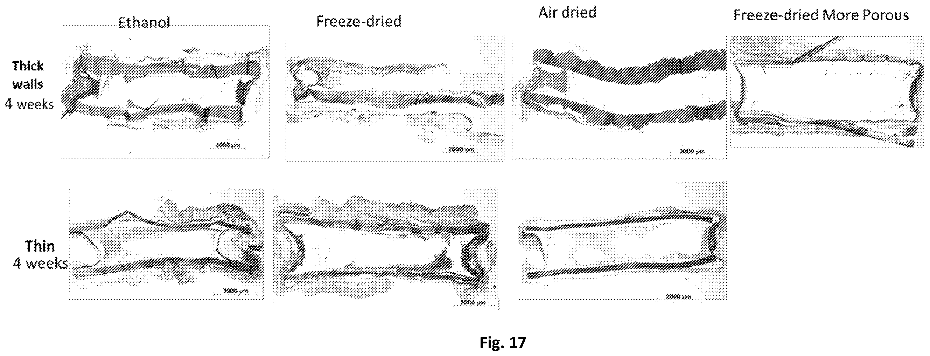

Figure 17. Schematic representation of: Longitudinal view of thick and thin wall conduits after 4 weeks in vivo subcutaneous implantation in mice.

### Detailed description

The present invention relates to a silk fibroin tubular conduit, and to a composition of silk hydrogel enzymatically cross-linked for use in the treatment of peripheral nerve injury, a spinal cord injury, regeneration of peripheral nerve cells, brain diseases or nerve degenerative diseases, in particular for the use for peripheral nerve regeneration.

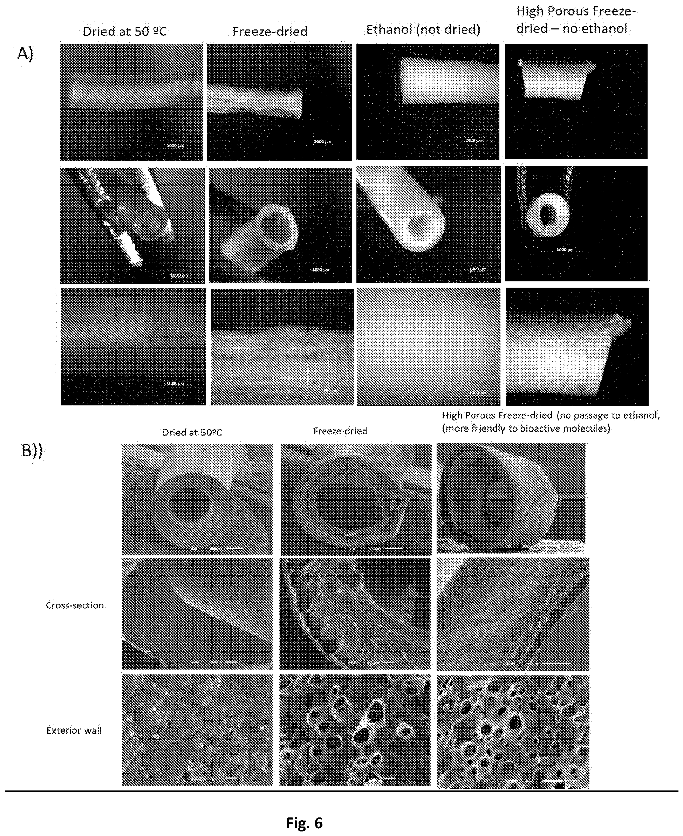

In an embodiment, by changing the method of solvent extraction, diverse conduits can be obtained, that will implicate changes in all features of the conduits (see fig. 6).

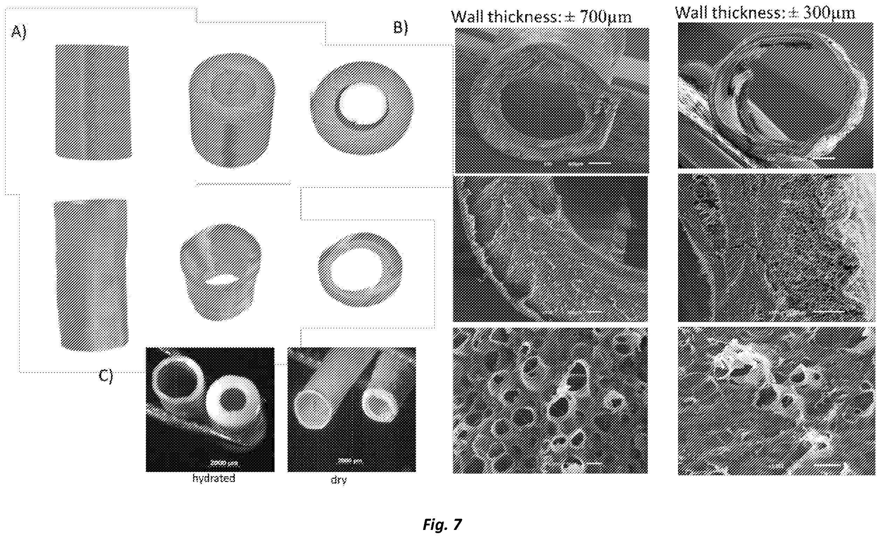

In an embodiment, the mold used to inject the silk polymeric solution has a crucial impact on the size specifications of the final conduit, which in other hand, it has an impact on the conduits characteristics. Several different wall thicknesses may be obtained, as seen in fig. 7.

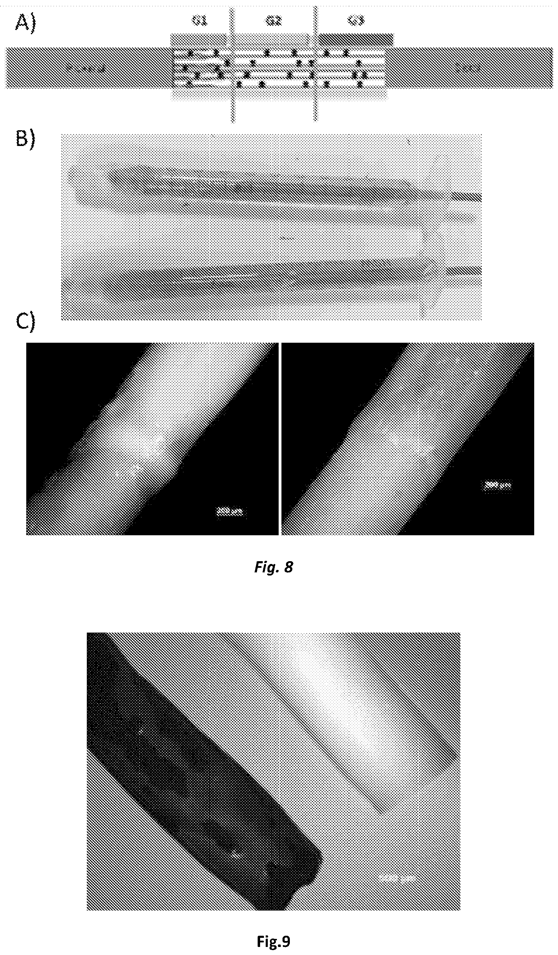

In an embodiment, tubular/nerve conduit of the present disclosure may have different drugs/bioactive/conductive molecules and/or a random drug distribution in the tube sections. In particular (see fig 8):
(I) Growth factors gradient: Growth factors relevant to regeneration, such as GDNF, NGF, BDNF, FGF of VEGF can be incorporated. Since the lack of vascularization and nutrients supply in the distal site of injuries is a major complication leading to cell dead and there is the need to guide proximal growing axons to the distal site, one of the objective is to have higher amounts of growth factors in this distal area. Since in a middle step of forming the NGC we will be handling a viscous silk hydrogel, it is possible to make gradients of several molecules, increasing its concentration from proximal to the distal site, where it is most needed
(II) Conductive conduits: An important aspect of synthetic nerve grafts is their ability to conduct electricity. Studies have showed that electrical stimulation can significantly promote the regeneration of peripheral nerve injuries.
(III) Incorporation of proteins, in particular: as a method to increase biological properties, the inclusion of human hair keratin is very feasible and already proved to be effective, increasing cellular viability in the conduits containing only 1% of keratin in the polymeric solution.

In an embodiment, simple conduits are composed only of silk. However, since the main purpose of these biomaterials is for peripheral nerve regeneration applications, many different molecules can be added to the conduits, for varying purposes.

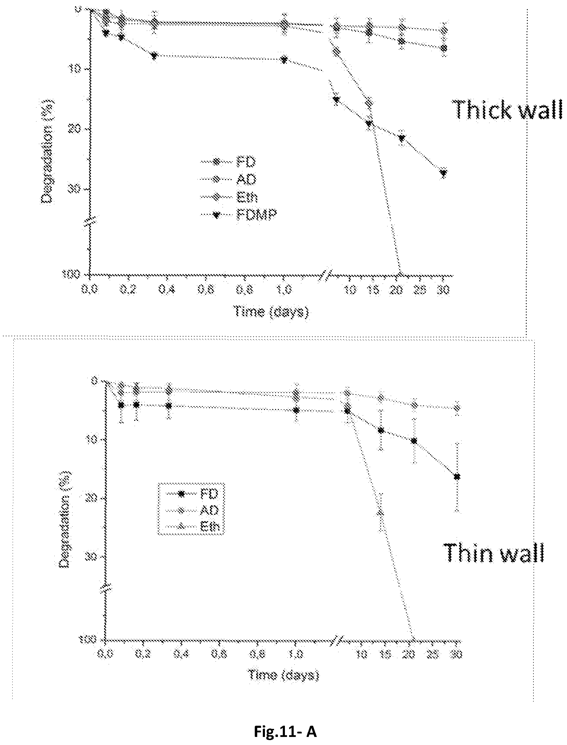

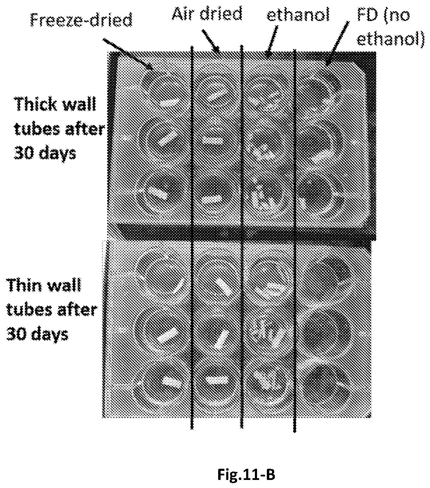

In an embodiment, different drying methods or simply not drying the silk conduits after the fabrication method affects all properties of the conduits. Degradation is one of these parameters that is largely affected. The conduits that are not dried, or used directly from ethanol, degrade much faster than the others (after 21 days) and tend to disaggregate in blocks in both thin and thick wall tubes (see fig 11-B). The freeze-dried formulations have an intermediate degradation, while the air dried formulations lasts for longer periods of time (only degrades 5% after 30 days). With these results is possible to verify that using the exact same methodology and concentration, and changing only the method of drying, we can tune the degradation from total degradation to almost not degrading after 30 days, according to needs.

In an embodiment, the drying of the conduits in different manners has implications in the physico-chemical characteristics of silk conduits. After 30 days in Simulated Body Fluid (SBF), the only formulation that presented bioactivity capabilities or that allowed the formation of calcified crystals, was the air dried formulation. That was proved by EDS technique, which detected a high concentration of calcium phosphates (ions phosphor and calcium) and by SEM, which detected these crystals in the surface of the conduit. For peripheral nerve regeneration purposes, calcification is not desired. However, this feature can be of high importance in hard tissue regeneration applications, such as bone regeneration.

In an embodiment, nerve conduits must not collapse and retain mechanical stability to withstand the traction of moving joints. Peripheral nerves are under tensile loads in situ and experience ~11% strain in resting position. Concerning this strain, thick tubes tolerate a higher tensile stress when compared to thinner tubes. Among the thick, FDMP and AD present higher tensile stress for the same % of strain. Regarding the tensile modulus, corroborating the previous results, thick tubes present higher modulus. Again, the FDMP and air dried revealed higher stiffness when compared to others. Corroborating the previous results, the air dried formulation, due to bioactivity and stiffness could easily find application in hard tissue regeneration applications.

In an embodiment, depending on the application of silk conduits, more or less permeability is an important feature to consider. In the case of peripheral nerve regeneration, it is important to keep permeability to oxygen and nutrients for the regenerating nerve, however maintaining a close and protective environment. Therefore, permeability assays are of outmost importance. By simply changing the method of drying or the thickness of the conduit walls, different permeability is obtained. In a 48h assay, a fluorescent molecule (fitc-dextran with 4kDa of weight) was injected in the lumen of the conduits and the ends were sealed. The release of this molecule through the conduit walls was evaluated. In thick wall conduits, only ethanol formulation allows 80% passage of molecule. On the other hand, all thin wall tubes allow the 4kDa molecule to cross through the walls, regardless of the formulation, reaching 100% of release after 48H. The microscopic images of the conduits after the assay suggest some molecules are entrapped in the thick wall conduits.

In an embodiment, the wall conduits varied from ±700 µm in thick wall conduits to ±300 µm in thin wall conduits, which made a significant difference in terms of permeability. For instance, in the air dried formulation, the release went from 20% to 100%, due to the wall thickness alone.

In an embodiment, the cytocompatibility and in vitro biological performance was carried out by means of using different cell types relevant for peripheral nerve regeneration, namely Human Schwann cells and human skin fibroblasts (BJ). All formulations of conduits were tested. Quantitative results of cellular density after 7 days in culture are shown (Alamar Blue assay) and corroborated by qualitative images (Live/dead assay).

In an embodiment, the silk conduits formulations of the present disclosure were implanted in male CD1 mice, in four subcutaneous pockets on the animals' backs. Animals were sacrificed 4 weeks after implantation, and conduits were explanted with the surrounding connective tissue for analysis. Sections were prepared and stained with hematoxylin and eosin (H&E). It is possible to see the expected formation of a fibrous capsule around the conduits. However that tissue does not show a high concentration of inflammatory cells, indicating that the host response to the silk conduit was negligible and in agreement with prior findings on the host response to silk fibroin biomaterials. It is also possible to see that there is no infiltration or migration of cells through the conduits walls, proving the conduits impermeability to undesired cells such as fibroblasts and consequent formation of scar tissue.

**Table 1 - Commercially available nerve guides and wraps. Apart from Avance, Qigel and RevolNerve, all other listed nerve devices are FDA approved.**

| Commercial name | Company | Material |
|---|---|---|
| Neuragen^{®}/Neurawrap^{™} | Integra | Type I collagen |
| Neurolac^{®} | Polyganics | PDLLA/CL |
| Neurotube^{®} | Synovis | PGA |
| Neuromatrix/Neuroflex^{™} conduits and NeuroMend^{™} wrap | Collagen matrix Inc. | Type I collagen |
| Salubridge^{™}/Salutunnel^{™} | Salumedica | Polyvinyl alcohol hydrogel |
| Surgisis^{®}/Axoguard^{™} | AxoGenlnc | Porcine small intestine submucosa |
| Avance^{®} | AxoGenlnc | Decellularized nerve |
| QiGel^{™}, Re-Axon^{®} | Medovent | Chitosan |
| RevolNerve^{®} | Orthomed | Collagen type I and III from porcine skin |

In an embodiment, silk fibroin from the silk worm Bombyx mori, has often been used as a textile material, yet, more and more attention has been given to silk lately due to its appropriate processing, biodegradability and the presence of easy accessible chemical groups for functional modifications. Studies suggest that silk is not only biodegradable but also bioresorbable, characteristics for tissue engineering and regenerative medicine. The major advantage of silk compared to other natural biopolymers is its excellent mechanical property. Other important advantages include good biocompatibility, water based processing, biodegradability and the presence of easy accessible chemical groups for functional modifications. Studies suggest that silk is not only biodegradable but also bioresorbable.

In an embodiment, regarding the application of this biomaterial in peripheral nerve regeneration, it is known that silk fibroin supports the viability of dorsal root ganglia and Schwann cells without affecting their normal phenotype or functionality.

The present disclosure refers to the development of a nerve guidance conduit derived from silk fibroin, using a different methodology than the ones referred to in the literature, intended for bridging nerve defects, for peripheral nerve regeneration.

In an embodiment, the porosity of the tube wall may be defined by the drying method. For peripheral nerve regeneration, it is necessary to have some porosity that allows the exchange of oxygen and nutrients from the outside to the region of the lesion. However, such porosity must block the infiltration of cells, such as fibroblasts that will induce fibrosis and scar tissue formation. In addition, tubes frozen with liquid nitrogen presented cracks and are more brittle, for which they were excluded.

In an embodiment, the tubes that were not dried and were used directly after being soaked in ethanol cannot be observed by SEM.

In an embodiment, the kink resistance ability was determined by bending the conduits 180 º on a flexible wire. The thick wall formulations that were produced by inducing β-sheet conformation with ethanol present kink resistance ability, with no occlusion of the lumen whatsoever. This feature is important, since nerves are close to articulations and might be subjected to forces. The device used must be capable of bending during patient movement without kinking and compression.

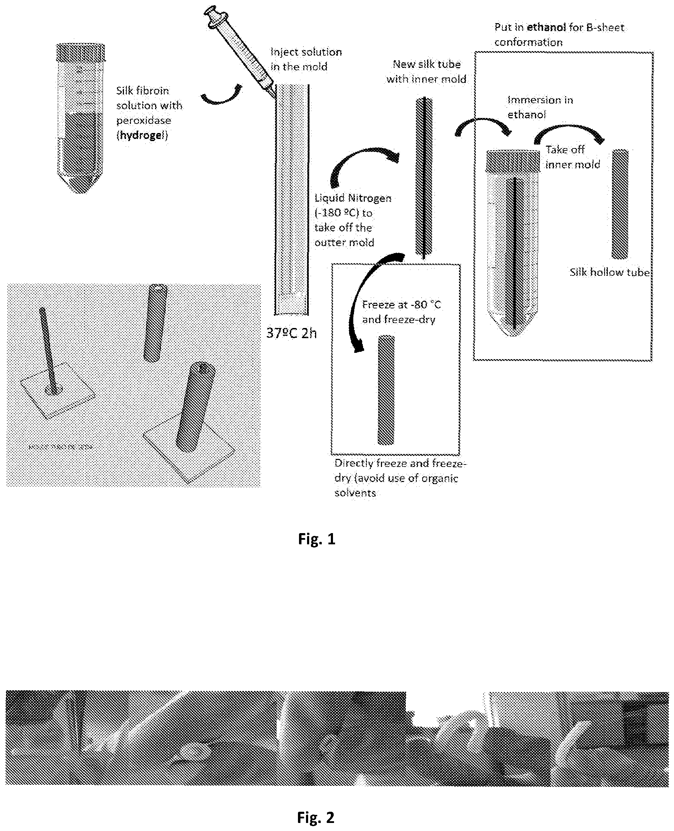

In an embodiment, the tubular conduit can be obtainable by the following steps:
- preparing an aqueous silk fibroin (SF) solution - concentration to be defined according to final intended features;
- adding of horseradish peroxidase (100 µL/ml of silk solution) and hydrogen peroxide (65 µL/ml of silk solution) - quantity to be defined according to final intended features;
- injecting the enzymatically cross-linked SF hydrogel into the mould (with the preferred dimensions);
- incubating the whole system at 37 °C for 2 hours for the complete formation of the hydrogel;
- placing the whole system in liquid nitrogen (-190 °C) for 30-45 seconds, until temporary development of β-sheet (with low temperature);
- the induction of β-sheet conformation by freezing at -80 °C and freeze-drying may be performed to avoid the use of organic solvents;
- For the methodology involving the β-sheet conformation using organic solvents such as ethanol, after placing the whole system in liquid nitrogen (-190 °C) for 30-45 seconds
   - removing outer mould;
   - placing in ethanol absolute for 1 hour for induction of β-sheet conformation;
   - removing the inner mould;
   - placing in water (Figure 2);
- proceed with most suitable method of drying according to the final requirements.

Aqueous solutions of silk fibroin with different concentrations work as precursors for the formation of the hydrogel. The silk fibroin solutions are capable of forming hydrogels in the presence of horseradish peroxidase and hydrogen peroxide (oxidizer) at mild temperatures and within physiological pH range. During the gelation procedure, it is allowed the combination of bioactive reagents, detecting reagents and any combination thereof. At this stage and in order to tackle peripheral nerve regeneration difficulties such as excessive fibrosis and scar tissue formation or to stimulate vascularization, we can include anti-fibrotic substances, angiogenic growth factors such as VEGF or even neurotrophic growth factors such as NGF and GDNF. The blending of silk with other polymers to enhance mechanical properties or cell adhesion such as keratin is also feasible. Later on this process, and its innovation, resides in the rest of the process and the additional steps necessary until the obtainment of the final nerve guidance conduit. We also envision the incorporation of conductive and drug-delivery nanoparticles, as gold nanoparticles. These have the capability of delivering agents of interest in the case of peripheral nerve regeneration and guide electrical impulses, since electrical stimulation is a proved method to improve regeneration, so by this approach we can have two advantages in the addition of one component.

The physicochemical and biological performances of the silk nerve guidance conduit (e.g., compressive modulus, storage modulus, stiffness, swelling behaviour, durability, degradation profile, porosity, permeability, suture ability) can be tuned for specific uses, by means of using different drying methods, different sized moulds and concentrations of silk fibroin in the same process. Furthermore, the inner diameter, thickness of the wall and length of the nerve guidance conduit can be tuned according to the final needs of the user and depends solely on the moulds used. According to the final objectives (animal model and size of nerve defect), different sizes are needed and can easily be obtained.

In an embodiment, with this methodology, it is possible to fine-tune the permeability of the nerve guidance conduit, in particular, by controlling the porosity of the tube wall.

**Table II - Pore size/ Drying method**

| | Pore size (µm) |
|---|---|
| Dried at 50 °C (passage in ethanol) | 0 ± 3 µm |
| Frozen at -80 °C and freeze dried (passage in ethanol) | 1.5 ± 0.5 µm |
| Frozen with liquid N₂ and freeze dried (passage in ethanol) | 0.9 ± 0.3 µm |
| Frozen at -80 °C and freeze dried (no passage in ethanol) | 2 ± 0.5 µm |

The simplicity of a regular mold casting or dipping technique does not allow for fine control over conduits wall thickness, uniformity and pore size or distribution. We were able to refine this mold casting method by introducing an extra step in the process: the formation of a hydrogel. This additional cross-linking step leads to a stronger and more stable three-dimensional network, thus conferring the scaffold higher mechanical properties, more elasticity and a lower degradation rate, when compared to tubes that did not undergo this step before turning in β-sheet conformation.

The term "comprising" whenever used in this document is intended to indicate the presence of stated features, integers, steps, components, but not to preclude the presence or addition of one or more other features, integers, steps, components or groups thereof.

It will be appreciated by those of ordinary skill in the art that unless otherwise indicated herein, the particular sequence of steps described is illustrative only and can be varied without departing from the disclosure. Thus, unless otherwise stated the steps described are so unordered meaning that, when possible, the steps can be performed in any convenient or desirable order.

Reference is made to the following documents:
1. Oh SH, Kim JH, Song KS, Jeon BH, Yoon JH, Seo TB, et al. Peripheral nerve regeneration within an asymmetrically porous PLGA/Pluronic F127 nerve guide conduit. Biomaterials. 2008 Apr;29(11):1601-9. PubMed PMID: 18155135.
2. Belkas JS, Shoichet MS, Midha R. Peripheral nerve regeneration through guidance tubes. Neurological research. 2004 Mar;26(2):151-60. PubMed PMID: 15072634
3. Ichihara S, Inada Y, Nakamura T. Artificial nerve tubes and their application for repair of peripheral nerve injury: an update of current concepts. Injury. 2008 Oct;39 Suppl 4:29-39. PubMed PMID: 18804584.
4. Johnson EO, Soucacos PN. Nerve repair: experimental and clinical evaluation of biodegradable artificial nerve guides. Injury. 2008 Sep;39 Suppl 3:S30-6. PubMed PMID: 18722612.
5. Yang Y, Chen X, Ding F, Zhang P, Liu J, Gu X. Biocompatibility evaluation of silk fibroin with peripheral nerve tissues and cells in vitro. Biomaterials. 2007 Mar;28(9):1643-52. PubMed PMID: 17188747.

## Claims

1. Tubular conduit comprising an enzymatically cross-linked silk fibroin hydrogel, wherein said tube is predominantly β-sheet conformation and comprises a porosity from 1-70% and a pore size from 1-10 µm.

2. Tubular conduit according to the previous claim comprising a pore size between 1-7 µm.

3. Tubular conduit according to any of the previous claims wherein the tubular conduit has a wall with a thickness of 0.2 mm - 2 mm, preferably 0.3 mm - 1 mm, more preferably 0.3 mm - 0.7 mm.

4. Tubular conduit according to any of the previous claims further comprising a conductive agent, preferably selected from a list consisting of: gold, silver, polypirrole, or mixtures thereof.

5. Tubular conduit according to any of the previous claims wherein the cross-linked silk fibroin hydrogel is enzymatically cross-linked with horseradish peroxidase and hydrogen peroxide.

6. Tubular conduit according to any of the previous claims wherein said hydrogel comprises between 5-25% (wt %) of silk fibroin.

7. Tubular conduit according to any of the previous claims wherein said hydrogel comprises between 12-18% (wt %) of silk fibroin, preferably 15-16% of silk fibroin.

8. Tubular conduit according to any of the previous claims wherein the porosity is of 1 - 50%, preferably 3-25%, more preferably 5-10%.

9. Tubular conduit according to any of the previous claims wherein the tubular conduit has an internal diameter of 1 mm -10 mm, preferably 2 mm - 4 mm.

10. Tubular conduit according to any of the previous claims wherein the tubular conduit has a length of 5 mm - 100 mm, preferably 10 mm - 50 mm.

11. Tubular conduit according to any of the previous claims wherein the tubular conduit further comprises hyaluronic acid, alginate, casein, polyethylene oxide, polyethylene glycol, collagen, fibronectin, keratin, polyaspartic acid, polylysine, chitosan, pectin, polylactic acid, polycaprolactone, polyglycolic acid, polyhydroxyalkanoate, polyanhydride, and mixtures thereof.

12. Tubular conduit according to any of the previous claims wherein the tubular conduit further comprises a biologically active agent, a therapeutic agent, an additive, a pharmaceutically acceptable excipient, a pharmaceutically acceptable carrier, and mixtures thereof, preferably wherein the biologically active agent is selected from the following list: a peptide, a protein, a nucleic acid, an antibody, an aptamer, an anticoagulant agent, a growth factor, a cytokine, an antibiotic, an immunosuppressor, a steroid, non-steroidal anti-inflammatory drug, and mixtures thereof.

13. Tubular conduit according to any of the previous claims wherein the tubular conduit comprises of dorsal root ganglia, Schwann cells and/or stem cells.

14. A composition of silk fibroin hydrogel enzymatically cross-linked, in particular with horseradish peroxidase and hydrogen peroxide,
for use in the treatment of peripheral nerve injury, a spinal cord injury, regeneration of peripheral nerve cells, brain diseases or nerve degenerative diseases;
wherein said composition is administrated in the form of a tubular conduit; wherein said tubular conduit comprises a β-sheet conformation obtainable by an enzymatically cross-linked of silk fibroin hydrogel; comprising a porosity from 1 to 70% and a pore size from 1 to 10 µm.

15. Composition according to the previous claim for use in the treatment of traumatic peripheral nerve injury.

## Patentansprüche

1. Röhrenförmiger Kanal, umfassend ein enzymatisch vernetztes Seidenfibroin-Hydrogel, wobei der genannte Kanal überwiegend ein β-Faltblatt Konformation, eine Porosität von 1-70 % und eine Porengröße von 1-10 µm aufweist.

2. Röhrenförmiger Kanal nach dem vorangehenden Anspruch mit einer Porengröße zwischen 1-7 µm.

3. Röhrenförmiger Kanal nach einem der vorangehenden Ansprüche, wobei der röhrenförmige Kanal eine Wand mit einer Stärke von 0,2 mm - 2 mm, bevorzugt 0,3 mm - 1 mm, besonders bevorzugt 0,3 mm - 0,7 mm aufweist.

4. Röhrenförmiger Kanal nach einem der vorangehenden Ansprüche, ferner umfassend ein leitfähiges Mittel, das bevorzugt aus einer Liste ausgewählt wird, bestehend aus: Gold, Silber, Polypyrrol oder Mischung davon.

5. Röhrenförmiger Kanal nach einem der vorangehenden Ansprüche, wobei das vernetzte Seidenfibroin-Hydrogel enzymatisch mit Meerrettichperoxidase und Wasserstoffperoxid vernetzt ist.

6. Röhrenförmiger Kanal nach einem der vorangehenden Ansprüche, wobei das genannte Hydrogel zwischen 5-25 % (wt %) Seidenfibroin enthält.

7. Röhrenförmiger Kanal nach einem der vorangehenden Ansprüche, wobei das genannte Hydrogel zwischen 12-18% (wt%) Seidenfibroin, bevorzugt 15-16% Seidenfibroin enthält.

8. Röhrenförmiger Kanal nach einem der vorangehenden Ansprüche, wobei die Porosität 1-50 %, bevorzugt 3-25 %, besonders bevorzugt 5-10% beträgt.

9. Röhrenförmiger Kanal nach einem der vorangehenden Ansprüche, wobei der röhrenförmige Kanal einen Innendurchmesser von 1 mm - 10 mm, bevorzugt 2 mm - 4 mm aufweist.

10. Röhrenförmiger Kanal nach einem der vorangehenden Ansprüche, wobei der röhrenförmige Kanal eine Länge von 5 mm - 100 mm, bevorzugt 10 mm - 50 mm aufweist.

11. Röhrenförmiger Kanal nach einem der vorangehenden Ansprüche, wobei der röhrenförmige Kanal ferner Hyaluronsäure, Alginat, Kasein, Polyethylenoxid, Polyethylenglykol, Kollagen, Fibronektin, Keratin, Polyasparaginsäure, Polylysin, Chitosan, Pektin, Polymilchsäure, Polycaprolacton, Polyglykolsäure, Polyhydroxyalkanoat, Polyanhydrid und Mischungen davon umfasst.

12. Röhrenförmiger Kanal nach einem der vorangehenden Ansprüche, wobei der röhrenförmige Kanal ferner ein biologisch aktives Mittel, ein Arzneimittel, einen Zusatzstoff, einen pharmazeutisch verträglichen Hilfsstoff, einen pharmazeutisch verträglichen Träger und Mischungen davon umfasst, wobei das biologisch aktive Mittel bevorzugt aus folgender Liste ausgewählt wird: ein Peptid, ein Protein, eine Nukleinsäure, ein Antikörper, ein Aptamer, ein gerinnungshemmendes Mittel, ein Wachstumsfaktor, ein Zytokin, ein Antibiotikum, ein Immunsuppressivum, ein Steroid, ein nichtsteroidaler Entzündungshemmer und Mischungen davon.

13. Röhrenförmiger Kanal nach einem der vorangehenden Ansprüche, wobei der röhrenförmige Kanal Spinalganglionien, Schwann-Zellen und/oder Stammzellen umfasst.

14. Eine Zusammensetzung aus enzymatisch vernetztem Seidenfibroin-Hydrogel, insbesondere mit Meerrettichperoxidase und Wasserstoffperoxid,
zur Verwendung bei der Behandlung von Verletzung peripherer Nerven, Verletzungen des Rückenmarks, Regeneration peripherer Nervenzellen, Erkrankungen des Gehirns oder neurodegenerativer Erkrankungen;
wobei die genannte Zusammensetzung in Form eines röhrenförmigen Kanals verabreicht wird;
wobei der genannte röhrenförmige Kanal eine β-Faltblatt Konformation umfasst, die über ein enzymatisch vernetztes Seidenfibroin-Hydrogel erhältlich ist; mit einer Porosität von 1 bis 70 % und einer Porengröße von 1 bis 10 µm.

15. Zusammensetzung nach dem vorangehenden Anspruch zur Verwendung bei der Behandlung von traumatischen Verletzungen peripherer Nerven.

## Revendications

1. Conduit tubulaire comprenant un hydrogel de fibroïne de soie enzymatiquement réticulé, dans lequel ledit tube est principalement en conformation de feuille beta et comprend une porosité de 1-70% et une taille de pore de 1-10 µm.

2. Conduit tubulaire selon la revendication précédente comprenant une taille de pore entre 1-7 µm.

3. Conduit tubulaire selon l'une quelconque des revendications précédentes, dans lequel le conduit tubulaire a un mur avec une épaisseur de 0,2 mm - 2 mm, préférablement 0,3 mm - 1 mm, plus préférablement 0,3 mm - 0,7 mm.

4. Conduit tubulaire selon l'une quelconque des revendications précédentes comprenant également un agent conducteur, préférablement sélectionné à partir d'une liste consistant en: or, argent, polypyrrole, ou des mélanges de ceux-ci.

5. Conduit tubulaire selon l'une quelconque des revendications précédentes, dans lequel l'hydrogel de fibroïne de soie réticulé est enzymatiquement réticulé avec de la peroxydase de raifort et du peroxyde d'hydrogène.

6. Conduit tubulaire selon l'une quelconque des revendications précédentes, dans lequel ledit hydrogel comprend entre 5-25% (% poids) de fibroïne de soie.

7. Conduit tubulaire selon l'une quelconque des revendications précédentes, dans lequel ledit hydrogel comprend entre 12-18% (% poids) de fibroïne de soie, préférablement 15-16% de fibroïne de soie.

8. Conduit tubulaire selon l'une quelconque des revendications précédentes, dans lequel la porosité est de 1-50%, préférablement 3-25%, plus préférablement 5-10%.

9. Conduit tubulaire selon l'une quelconque des revendications précédentes, dans lequel le conduit tubulaire a un diamètre interne de 1 mm - 10 mm, plus préférablement 2 mm - 4 mm.

10. Conduit tubulaire selon l'une quelconque des revendications précédentes, dans lequel le conduit tubulaire a une longueur de 5 mm - 100 mm, préférablement 10 mm - 50 mm.

11. Conduit tubulaire selon l'une quelconque des revendications précédentes, dans lequel le conduit tubulaire comprend également de l'acide hyaluronique, de l'alginate, de la caséine, de l'oxyde de polyéthylène, du polyéthylène glycol, du collagène, de la fibronectine, de la kératine, de l'acide polyaspartique, de la polylysine, du chitosan, de la pectine, de l'acide polylactique, de la polycaprolactone, de l'acide polyglycolique, du polyhydroxyalcanoate, du polyanhydride, et des mélanges de ceux-ci.

12. Conduit tubulaire selon l'une quelconque des revendications précédentes, dans lequel le conduit tubulaire comprend également un agent biologiquement actif, un agent thérapeutique, un additif, un excipient pharmaceutiquement acceptable, un support pharmaceutiquement acceptable, et des mélanges de ceux-ci, préférablement dans lequel l'agent biologiquement actif est sélectionné à partir de la liste suivante: un peptide, une protéine, un acide nucléique, un anticorps, un aptamère, un anticoagulant, un facteur de croissance, une cytokine, un antibiotique, un immunosuppresseur, un stéroïde, un médicament anti-inflammatoire non stéroïdien, et des mélanges de ceux-ci.

13. Conduit tubulaire selon l'une quelconque des revendications précédentes, dans lequel le conduit tubulaire est composé de ganglions rachidiens, cellules de Schwann et/ou cellules souche.

14. Une composition d'hydrogel de fibroïne de soie enzymatiquement réticulé, en particulier avec de la peroxydase de raifort et du peroxyde d'hydrogène,
pour une utilisation dans le traitement d'une lésion nerveuse périphérique, d'une lésion de la moelle épinière, la régénération de cellules cérébrales périphériques, les maladies cérébrales ou les maladies dégénératives nerveuses ;
dans lequel ladite composition est administrée sous forme d'un conduit tubulaire;
dans lequel ledit conduit tubulaire comprend une conformation de feuille beta pouvant être obtenue à travers un hydrogel de fibroïne de soie enzymatiquement réticulé; comprenant une porosité de 1 à 70% et une taille de pore de 1 à 10 µm.

15. Composition selon la revendication précédente pour une utilisation dans le traitement d'une lésion nerveuse périphérique traumatique.
